# EUROPEAN PATENT APPLICATION

(11) **EP 4 740 960 A1**
(43) Date of publication of application: **13.05.2026**
(21) Application number: 24838668.2
(22) Date of filing: 03.07.2024
(51) Int. Cl.: A61K 45/00, A61K 48/00, C12N 15/113, A61K 38/02

(54) **DRUG FOR TREATING TUMOR HAVING TP53 GENE MUTATION**

(30) Priority: 07.07.2023 CN 202310830989
(71) Applicant: Center for Excellence in Molecular Cell Science, Chinese Academy of Sciences, Shanghai 200031 (CN); Cytosinlab Therapeutics Co., Ltd., Shanghai 201203 (CN)
(72) Inventor: XU, Guoliang, Shanghai 200031 (CN); WU, Haiping, Shanghai 201203 (CN)
(74) Representative: Lavoix
(86) International application number: PCT/CN2024/103426
(87) International publication number: WO 2025/011413

(57) **Abstract**

Disclosed in the present invention is a drug for treating a tumor having a TP53 gene mutation. The drug comprises a thymine DNA glycosidase (TDG for short, the same below) inhibitor and a pharmaceutically acceptable auxiliary component. The TDG inhibitor used in the present invention can effectively inhibit human TDG protein expression and thus has a good treatment effect on a tumor having a TP53 gene mutation.

## Description

### TECHNICAL FIELD

The present invention relates to the field of medicine, specifically, the present invention discloses a drug for treating tumor having TP53 gene mutation.

### BACKGROUND

The accumulation of genetic and epigenetic changes in cells can lead to uncontrolled growth and proliferation, which in turn can trigger cancer. TP53 is one of the most common mutant genes in human cancer, and TP53 gene mutation occurs in approximately 50% of human cancers, comprising lung cancer, breast cancer, colon cancer, ovarian cancer and gastric cancer. TP53 mutation can occur at any stage of cancer development and is associated with poor prognosis and resistance to treatment.

TP53 is an important tumor suppressor gene that regulates the expression of genes related to cell cycle, DNA repair, and apoptosis as a transcription factor. Its mutation can lead to the loss of related protein function, resulting in the inability to induce cell apoptosis or arrest cell cycle in response to DNA damage, ultimately promoting tumor growth and metastasis. TP53 mutation can also lead to abnormal protein function, thereby promoting tumor growth and metastasis.

Although TP53 mutation is important in cancer development and progression, there are currently no drugs that directly target TP53 mutation. There are several challenges in developing direct targeted therapy for TP53, mainly reflected in the following aspects: (1) TP53 is a complex protein with multiple functions, making it difficult to develop drugs that selectively target mutant protein forms without affecting normal protein forms; (2) TP53 mutation can occur at any stage of cancer development, making it difficult to develop drugs that are effective for all stages of cancer; (3) TP53 mutation can be heterozygous or homozygous, resulting in different levels of protein expression and function, and this heterogeneity may make it difficult to develop effective drugs for all TP53 mutant tumors.

Although TP53 is a difficult target to drug, its genetic mutations not only cause it to lose normal function, but also activate cascade signaling pathways to promote tumor progression and compensate for the loss of function, suggesting that there may be a large number of "synthetic lethal" partners hidden in these altered pathways. In summary, there is still a need to develop tumor therapeutic drugs targeting TP53 in this field.

### SUMMARY OF THE INVENTION

The inventor has found through research that thymine DNA glycosylation enzyme (TDG for short, the same below) is a good "synthetic lethal" partner for TP53 gene mutations, and has great potential in the treatment of TP53 mutant tumors. The use of a pharmaceutical composition that comprises thymidine DNA glycosylation enzyme (TDG) inhibitors and pharmaceutically acceptable excipients thereof can effectively inhibit the expression of human TDG protein and have a good therapeutic effect on tumors having TP53 gene mutations.

In the first aspect of the present invention, it provides a use of a TDG inhibitor for the preparation of a pharmaceutical composition for the treatment of tumors associated with TP53 gene mutations;
wherein the pharmaceutical composition comprises a therapeutically effective amount of the TDG inhibitor and pharmaceutically acceptable excipients thereof.

In another preferred embodiment, the pharmaceutical composition is used to downregulate the expression level or expression amount of the TP53 gene in a subject.

In another preferred embodiment, the tumors associated with TP53 gene mutations are selected from the group consisting of: lung cancer, skin melanoma, liver cancer, breast cancer, acute myeloid leukemia, colon cancer, colorectal cancer, esophageal cancer, gastric cancer, pancreatic cancer, and bladder cancer.

In another preferred embodiment, the TP53 gene mutations are selected from the group consisting of: missense mutation, gene deletion, frameshift mutation, splice site mutation, and truncation mutation.

In another preferred embodiment, the protein sequence changes caused by TP53 gene mutations include but are not limited to the following groups:
p.146_149WVDS>C, p.157_158insL, p.165_166insYKQ, p.173_174VR>G, p.239_240insN, p.247_248NR>KW, p.249_250insR, p.A119fs, p.A138P, p.A159D, p.A159P, p.A159V, p.A161D, p.A161T, p.A189A, p.A347P, p.A347V, p.A70A, p.A74S, p.A76fs, p.A88fs, p.C124*, p.C124R, p.C135F, p.C135fs, p.C135R, p.C135Y, p.C141W, p.C141Y, p.C176F, p.C176fs, p.C176S, p.C176W, p.C176Y, p.C229fs, p.C238F, p.C238fs, p.C238G, p.C238R, p.C238S, p.C238Y, p.C242F, p.C242fs, p.C242G, p.C242R, p.C242S, p.C242W, p.C242Y, p.C275F, p.C275fs, p.C275G, p.C275Y, p.C277F, p.D148G, p.D207Y, p.D228fs, p.D259V, p.D259Y, p.D281G, p.D281N, p.D281Y, p.D48fs, p.D48N, p.D49H, p.E11Q, p.E171*, p.E171del, p.E180*, p.E180fs, p.E204*, p.E204fs, p.E224D, p.E224E, p.E224K, p.E258*, p.E258A, p.E258K, p.E271*, p.E271fs, p.E271K, p.E285K, p.E285V, p.E286K, p.E287*, p.E287D, p.E294*, p.E294fs, p.E298*, p.E326*, p.E326K, p.E336*, p.E336fs, p.E339*, p.E343*, p.E349*, p.E349fs, p.E51*, p.E56*, p.E62*, p.EE286del, p.F113C, p.F113L, p.F113S, p.F113V, p.F134fs, p.F341F, p.G105R, p.G108del, p.G108fs, p.G108V, p.G154V, p.G187C, p.G187R, p.G199*, p.G199G, p.G244A, p.G244C, p.G244D, p.G244S, p.G245C, p.G245D, p.G245R, p.G245S, p.G245V, p.G262D, p.G262S, p.G262V, p.G266*, p.G266E, p.G266R, p.G266V, p.G293fs, p.G334V, p.G356G, p.G360V, p.G389W, p.H178D, p.H179D, p.H179fs, p.H179Q, p.H179R, p.H179Y, p.H193fs, p.H193L, p.H193N, p.H193P, p.H193R, p.H193Y, p.H214H, p.H214R, p.H233del, p.H296Y, p.I162N, p.I195F, p.I195S, p.I195T, p.I232N, p.I232S, p.I251fs, p.I251L, p.I251N, p.I251T, p.I254D, p.I254S, p.I255del, p.I255N, p.I255S, p.I255T, p.K101*, p.K101fs, p.K120*, p.K120fs, p.K120N, p.K132E, p.K132M, p.K132N, p.K132Q, p.K132R, p.K132T, p.K139fs, p.K164*, p.K164E, p.K164N, p.K291*, p.K291E, p.K291L, p.K292I, p.K319*, p.K320N, p.K382fs, p.L130F, p.L130R, p.L130V, p.L137Q, p.L145R, p.L194F, p.L194R, p.L252fs, p.L252L, p.L264R, p.L265P, p.L323fs, p.L35fs, p.L93fs, p.LAPP188del, p.M133K, p.M237I, p.M246I, p.M246R, p.M246T, p.M246V, p.N131K, p.N200fs, p.N235del, p.N239D, p.N239S, p.N268fs, p.N268I, p.N310fs, p.N345fs, p.N345S, p.P142fs, p.P142R, p.P151A, p.P151H, p.P151S, p.P152fs, p.P152L, p.P152P, p.P152S, p.P153fs, p.P177L, p.P177T, p.P190L, p.P191del, p.P191fs, p.P219fs, p.P223L, p.P250L, p.P278F, p.P278H, p.P278L, p.P278P, p.P278R, p.P278S, p.P278T, p.P27L, p.P295P, p.P301fs, p.P309S, p.P316P, p.P318fs, p.P322fs, p.P36fs, p.P36L, p.P47fs, p.P47P, p.P58fs, p.P98fs, p.P98L, p.P98S, p.PPQH190del, p.Q100*, p.Q104*, p.Q136*, p.Q136fs, p.Q144*, p.Q167*, p.Q16L, p.Q192*, p.Q317*, p.Q317fs, p.Q331*, p.Q331R, p.Q38*, p.Q52*, p.Q52fs, p.Q52P*, p.R110C, p.R110L, p.R110P, p.R156P, p.R158G, p.R158H, p.R158L, p.R158P, p.R174fs, p.R175fs, p.R175H, p.R175L, p.R181C, p.R181H, p.R181P, p.R196*, p.R196P, p.R209*, p.R209fs, p.R213*, p.R213G, p.R213L, p.R213Q, p.R248G, p.R248L, p.R248Q, p.R248W, p.R249fs, p.R249G, p.R249M, p.R249S, p.R249T, p.R263S, p.R267fs, p.R267L, p.R267P, p.R267Q, p.R267W, p.R273C, p.R273H, p.R273L, p.R280fs, p.R280G, p.R280K, p.R280R, p.R280S, p.R280T, p.R282G, p.R282Q, p.R282W, p.R283C, p.R283del, p.R283H, p.R283P, p.R290C, p.R290fs, p.R290H, p.R306*, p.R306fs, p.R335fs, p.R337C, p.R337L, p.R342*, p.R342fs, p.R379fs, p.S106R, p.S127C, p.S127F, p.S127P, p.S149fs, p.S166*, p.S183*, p.S185S, p.S215C, p.S215del, p.S215G, p.S215I, p.S215N, p.S215R, p.S240R, p.S241A, p.S241C, p.S241F, p.S241Y, p.S261T, p.S313fs, p.S315fs, p.S33fs, p.S37fs, p.S90F, p.S90fs, p.S94*, p.S95F, p.S99fs, p.T118fs, p.T125M, p.T125P, p.T125T, p.T140fs, p.T155N, p.T155P, p.T211A, p.T211I, p.T256fs, p.T284fs, p.V122fs, p.V143A, p.V143M, p.V147D, p.V147G, p.V157F, p.V157fs, p.V173L, p.V173M, p.V197fs, p.V197L, p.V203L, p.V216L, p.V216M, p.V217G, p.V218del, p.V218L, p.V272E, p.V272fs, p.V272L, p.V272M, p.V274C, p.V274D, p.V274F, p.V31I, p.V73fs, p.W146*, p.W53*, p.W91*, p.W91fs, p.Y103fs, p.Y107*, p.Y107fs, p.Y126*, p.Y126C, p.Y163C, p.Y163H, p.Y205C, p.Y205F, p.Y205fs, p.Y205H, p.Y220C, p.Y220D, p.Y220fs, p.Y234C, p.Y234D, p.Y234fs, p.Y234N, p.Y236*, p.Y236C, p.Y236N, p.Y236S, p.Y327fs.
* indicates a stop codon mutation.

In another preferred embodiment, the TDG inhibitor is an inhibitor targeting the human TDG (GENE ID: 6996).

In another preferred embodiment, the NCBI accession number for the human TDG mRNA sequence is NM_003211.6.

In another preferred embodiment, the NCBI accession number for the human TDG protein sequence is NP_003202.3.

In another preferred embodiment, the TDG inhibitor is selected from the group consisting of small molecule inhibitors, polypeptide drugs, sgRNA compositions targeting the human TDG for use in CRISPR-Cas9 gene knockout technology, siRNA targeting the human TDG sequence for use in RNA interference technology and combinations thereof, shRNA targeting the human TDG sequence for use in RNA interference technology and combinations thereof.

In another preferred embodiment, the TDG inhibitor is a small molecule inhibitor as described in US20160199381A1, US2022213070A1, and WO2022047288A1.

In another preferred embodiment, the TDG inhibitor is an sgRNA targeting the human TDG sequence for use in CRISPR-Cas9 gene knockout technology , and the sgRNA is selected from the group consisting of (each presented 5' to 3'):

| | | |
|---|---|---|
| 1 | sg-1: | GCTATAAAACTGGAAGTTAG |
| 2 | sg-2: | TGTATTTCAGAGGCTCCAAA |
| 3 | sg-3: | ACGAAATATGGACGTTCAAG |
| 4 | sg-4: | TGGGTCATCCACTGCCCATT |
| 5 | sg-5: | TGGCATAAACCCGGGACTAA |
| 6 | sg-6: | TTTGACCTACAGCTTGCCCA |
| 7 | sg-7: | GTTGAGAGCGTGGAGTTAAG |
| 8 | sg-8: | TGAGGTCCAGCTGAACCATA |
| 9 | sg-9: | GGGCATCATTACCCTGGACC |
| 10 | sg-10: | CTACCAGGGAAGTATGGTAT. |

In another preferred embodiment, the TDG inhibitor is an siRNA targeting the human TDG sequence for use in RNA interference technology, and the siRNA is selected from the group consisting of (each presented 5' to 3'):

| | | |
|---|---|---|
| 11 | si-1S: | GAACGAAAUAUGGACGUUCAA |
| 12 | si-1AS: | UUGAACGUCCAUAUUUCGUUC |
| 13 | si-2S: | GGACUUAAGAGAUCAGUUGAA |
| 14 | si-2AS: | UUCAACUGAUCUCUUAAGUCC |
| 15 | si-3A: | GGUUAUGAGGCAGCAUAUGGU |
| 16 | si-3AS: | ACCAUAUGCUGCCUCAUAACC |
| 17 | si-4A: | CAGCUAUUCCCUUCAGCAA |
| 18 | si-4AS: | UUGCUGAAGGGAAUAGCUG |
| 19 | si-5A: | CCAGAACAACAGAACCAAA |
| 20 | si-5AS: | UUUGGUUCUGUUGUUCUGG |
| 21 | si-6A: | GGAUGAUCACACUCUACCA |
| 22 | si-6AS: | UGGUAGAGUGUGAUCAUCC |
| 23 | si-7A: | GCAAAGAUCUCUCCAGUAA |
| 24 | si-7AS: | UUACUGGAGAGAUCUUUGC |
| 25 | si-8A: | GAAGGAGGACGUAUUCUAGUA |
| 26 | si-8AS: | UACUAGAAUACGUCCUCCUUC |
| 27 | si-9A: | GACGUAUUCUAGUACAGAA |
| 28 | si-9AS: | UUCUGUACUAGAAUACGUC |
| 29 | si-10A: | CACGAAUAGCAGUGUUUAA |
| 30 | si-10AS: | UUAAACACUGCUAUUCGUG |
| 31 | si-11A: | GAAACUCUCUGCUAUGUUA |
| 32 | si-11AS: | UAACAUAGCAGAGAGUUUC |
| 33 | si-12A: | GACAAAGUUCAUUACUACA |
| 34 | si-12AS: | UGUAGUAAUGAACUUUGUC |
| 35 | si-13A: | GAAGGACUUAAGAGAUCAGUU |
| 36 | si-13AS: | AACUGAUCUCUUAAGUCCUUC |
| 37 | si-14A: | GACUUAAGAGAUCAGUUGA |
| 38 | si-14AS: | UCAACUGAUCUCUUAAGUC |
| 39 | si-15A: | GAAAGGCAUUGAACGAAAUAU |
| 40 | si-15AS: | AUAUUUCGUUCAAUGCCUUUC |
| 41 | si-16A: | GGACGUUCAAGAGGUGCAA |
| 42 | si-16AS: | UUGCACCUCUUGAACGUCC |
| 43 | si-17A: | GUUCAAGAGGUGCAAUAUA |
| 44 | si-17AS: | UAUAUUGCACCUCUUGAAC |
| 45 | si-18A: | GCAAUAUACAUUUGACCUA |
| 46 | si-18AS: | UAGGUCAAAUGUAUAUUGC |
| 47 | si-19A: | GGUGGUGCUUACGGAGAAA |
| 48 | si-19AS: | UUUCUCCGUAAGCACCACC |
| 49 | si-20A: | GGAGUUAAGAGGAGAAUCA |
| 50 | si-20AS: | UGAUUCUCCUCUUAACUCC |
| 51 | si-21A: | GACCAAAUUCCUUCCUUUA |
| 52 | si-21AS: | UAAAGGAAGGAAUUUGGUC; |

| | | |
|---|---|---|
| in the above nucleic acid sequences, U nucleotides and T nucleotides are interchangeable. | | |

In another preferred embodiment, the TDG inhibitor is an shRNA targeting the human TDG sequence for use in RNA interference technology, and the shRNA is selected from the group consisting of (each presented 5' to 3'):

| | | |
|---|---|---|
| 53 | sh-1A: | CCGGGAACGAAATATGGACGTTCAACTCGAGTTGA ACGTCCATATTTCGTTCTTTTTG |
| 54 | sh-1AS: | AATTCAAAAAGAACGAAATATGGACGTTCAACTC GAGTTGAACGTCCATATTTCGTTC |
| 55 | sh-2A: | CCGGGGACTTAAGAGATCAGTTGAACTCGAGTTC AACTGATCTCTTAAGTCCTTTTTG |
| 56 | sh-2AS: | AATTCAAAAAGGACTTAAGAGATCAGTTGAACTC GAGTTCAACTGATCTCTTAAGTCC |
| 57 | sh-3A: | CCGGGGTTATGAGGCAGCATATGGTCTCGAGACCA TATGCTGCCTCATAACCTTTTTG |
| 58 | sh-3AS: | AATTCAAAAAGGTTATGAGGCAGCATATGGTCTC GAGACCATATGCTGCCTCATAACC |

| | | |
|---|---|---|
| 59 | sh-4A: | CCGGCAGCTATTCCCTTCAGCAACTCGAGTTGCT GAAGGGAATAGCTGTTTTTG |
| 60 | sh-4AS: | AATTCAAAAACAGCTATTCCCTTCAGCAACTCGA GTTGCTGAAGGGAATAGCTG |
| 61 | sh-5A: | CCGGCCAGAACAACAGAACCAAACTCGAGTTTGGTT CTGTTGTTCTGGTTTTTG |
| 62 | sh-5AS: | AATTCAAAAACCAGAACAACAGAACCAAACTCGA GTTTGGTTCTGTTGTTCTGG |
| 63 | sh-6A: | CCGGGGATGATCACACTCTACCACTCGAGTGGTA GAGTGTGATCATCCTTTTTG |
| 64 | sh-6AS: | AATTCAAAAAGGATGATCACACTCTACCACTCGA GTGGTAGAGTGTGATCATCC |
| 65 | sh-7A: | CCGGGCAAAGATCTCTCCAGTAACTCGAGTTACTGG AGAGATCTTTGCTTTTTG |
| 66 | sh-7AS: | AATTCAAAAAGCAAAGATCTCTCCAGTAACTCGAG TTACTGGAGAGATCTTTGC |
| 67 | sh-8A: | CCGGGAAGGAGGACGTATTCTAGTACTCGAGTACTA GAATACGTCCTCCTTCTTTTTG |
| 68 | sh-8AS: | AATTCAAAAAGAAGGAGGACGTATTCTAGTACTCGAG TACTAGAATACGTCCTCCTTC |
| 69 | sh-9A: | CCGGGACGTATTCTAGTACAGAACTCGAGTTCTGTA CTAGAATACGTCTTTTTG |
| 70 | sh-9AS: | AATTCAAAAAGACGTATTCTAGTACAGAACTCGAG TTCTGTACTAGAATACGTC |
| 71 | sh-10A: | CCGGCACGAATAGCAGTGTTTAACTCGAGTTAAACA CTGCTATTCGTGTTTTTG |
| 72 | sh-10AS: | AATTCAAAAACACGAATAGCAGTGTTTAACTCGAG TTAAACACTGCTATTCGTG |
| 73 | sh-11A: | CCGGGAAACTCTCTGCTATGTTACTCGAGTAACA |

| | | |
|---|---|---|
| | | TAGCAGAGAGTTTCTTTTTG |
| 74 | sh-11AS: | AATTCAAAAAGAAACTCTCTGCTATGTTACTCGAG TAACATAGCAGAGAGTTTC |
| 75 | sh-12A: | CCGGGACAAAGTTCATTACTACACTCGAGTGTAGTA ATGAACTTTGTCTTTTTG |
| 76 | sh-12AS: | AATTCAAAAAGACAAAGTTCATTACTACACTCGAGTG TAGTAATGAACTTTGTC |
| 77 | sh-13A: | CCGGGAAGGACTTAAGAGATCAGTTCTCGAGAAC TGATCTCTTAAGTCCTTCTTTTTG |
| 78 | sh-13AS: | AATTCAAAAAGAAGGACTTAAGAGATCAGTTCTCGA GAACTGATCTCTTAAGTCCTTC |
| 79 | sh-14A: | CCGGGACTTAAGAGATCAGTTGACTCGAGTCAACTG ATCTCTTAAGTCTTTTTG |
| 80 | sh-14AS: | AATTCAAAAAGACTTAAGAGATCAGTTGACTCGAGT CAACTGATCTCTTAAGTC |
| 81 | sh-15A: | CCGGGAAAGGCATTGAACGAAATATCTCGAGATATTT CGTTCAATGCCTTTCTTTTTG |
| 82 | sh-15AS: | AATTCAAAAAGAAAGGCATTGAACGAAATATCTC GAGATATTTCGTTCAATGCCTTTC |
| 83 | sh-16A: | CCGGGGACGTTCAAGAGGTGCAACTCGAGTTGCAC CTCTTGAACGTCCTTTTTG |
| 84 | sh-16AS: | AATTCAAAAAGGACGTTCAAGAGGTGCAACTCGAG TTGCACCTCTTGAACGTCC |
| 85 | sh-17A: | CCGGGTTCAAGAGGTGCAATATACTCGAGTATATTG CACCTCTTGAACTTTTTG |
| 86 | sh-17AS: | AATTCAAAAAGTTCAAGAGGTGCAATATACTCGA GTATATTGCACCTCTTGAAC |
| 87 | sh-18A: | CCGGGCAATATACATTTGACCTACTCGAGTAGGTC AAATGTATATTGCTTTTTG |

| | | |
|---|---|---|
| 88 | sh-18AS: | AATTCAAAAAGCAATATACATTTGACCTACTCGA GTAGGTCAAATGTATATTGC |
| 89 | sh-19A: | CCGGGGTGGTGCTTACGGAGAAACTCGAGTTTCT CCGTAAGCACCACCTTTTTG |
| 90 | sh-19AS: | AATTCAAAAAGGTGGTGCTTACGGAGAAACTCGA GTTTCTCCGTAAGCACCACC |
| 91 | sh-20A: | CCGGGGAGTTAAGAGGAGAATCACTCGAGTGATT CTCCTCTTAACTCCTTTTTG |
| 92 | sh-20AS: | AATTCAAAAAGGAGTTAAGAGGAGAATCACTCGAG TGATTCTCCTCTTAACTCC |
| 93 | sh-21A: | CCGGGACCAAATTCCTTCCTTTACTCGAGTAAAGGA AGGAATTTGGTCTTTTTG |
| 94 | sh-21AS: | AATTCAAAAAGACCAAATTCCTTCCTTTACTCGAG TAAAGGAAGGAATTTGGTC. |

In another preferred embodiment, the pharmaceutical composition further comprises a second therapeutically active component that is an immune checkpoint inhibitor.

In another preferred embodiment, the immune checkpoint inhibitor is PD-1 monoclonal antibody.

In another preferred embodiment, the immune checkpoint inhibitor is CTLA-4 monoclonal antibody.

In the second aspect of the present invention, it provides a pharmaceutical combination comprising:
(1) a first therapeutically active component that is a TDG inhibitor;
(2) a second therapeutically active component that is an immune checkpoint inhibitor.

In the third aspect of the present invention, it provides an sgRNA targeting the human TDG sequence for use in CRISPR-Cas9 gene knockout technology, wherein the sgRNA is selected from the group consisting of (each presented 5' to 3'):

| | | |
|---|---|---|
| 1 | sg-1: | GCTATAAAACTGGAAGTTAG |
| 2 | sg-2: | TGTATTTCAGAGGCTCCAAA |
| 3 | sg-3: | ACGAAATATGGACGTTCAAG |
| 4 | sg-4: | TGGGTCATCCACTGCCCATT |
| 5 | sg-5: | TGGCATAAACCCGGGACTAA |
| 6 | sg-6: | TTTGACCTACAGCTTGCCCA |
| 7 | sg-7: | GTTGAGAGCGTGGAGTTAAG |
| 8 | sg-8: | TGAGGTCCAGCTGAACCATA |
| 9 | sg-9: | GGGCATCATTACCCTGGACC |
| 10 | sg-10: | CTACCAGGGAAGTATGGTAT. |

In the fourth aspect of the present invention, it provides an siRNA targeting the human TDG sequence, wherein the siRNA has a sequence selected from the group consisting of (each presented 5' to 3'):

| | | |
|---|---|---|
| 11 | si-1S: | GAACGAAAUAUGGACGUUCAA |
| 12 | si-1AS: | UUGAACGUCCAUAUUUCGUUC |
| 13 | si-2S: | GGACUUAAGAGAUCAGUUGAA |
| 14 | si-2AS: | UUCAACUGAUCUCUUAAGUCC |
| 15 | si-3A: | GGUUAUGAGGCAGCAUAUGGU |
| 16 | si-3AS: | ACCAUAUGCUGCCUCAUAACC |
| 17 | si-4A: | CAGCUAUUCCCUUCAGCAA |
| 18 | si-4AS: | UUGCUGAAGGGAAUAGCUG |
| 19 | si-5A: | CCAGAACAACAGAACCAAA |
| 20 | si-5AS: | UUUGGUUCUGUUGUUCUGG |
| 21 | si-6A: | GGAUGAUCACACUCUACCA |
| 22 | si-6AS: | UGGUAGAGUGUGAUCAUCC |
| 23 | si-7A: | GCAAAGAUCUCUCCAGUAA |
| 24 | si-7AS: | UUACUGGAGAGAUCUUUGC |
| 25 | si-8A: | GAAGGAGGACGUAUUCUAGUA |
| 26 | si-8AS: | UACUAGAAUACGUCCUCCUUC |
| 27 | si-9A: | GACGUAUUCUAGUACAGAA |
| 28 | si-9AS: | UUCUGUACUAGAAUACGUC |
| 29 | si-10A: | CACGAAUAGCAGUGUUUAA |
| 30 | si-10AS: | UUAAACACUGCUAUUCGUG |
| 31 | si-11A: | GAAACUCUCUGCUAUGUUA |
| 32 | si-11AS: | UAACAUAGCAGAGAGUUUC |
| 33 | si-12A: | GACAAAGUUCAUUACUACA |
| 34 | si-12AS: | UGUAGUAAUGAACUUUGUC |
| 35 | si-13A: | GAAGGACUUAAGAGAUCAGUU |
| 36 | si-13AS: | AACUGAUCUCUUAAGUCCUUC |
| 37 | si-14A: | GACUUAAGAGAUCAGUUGA |
| 38 | si-14AS: | UCAACUGAUCUCUUAAGUC |
| 39 | si-15A: | GAAAGGCAUUGAACGAAAUAU |
| 40 | si-15AS: | AUAUUUCGUUCAAUGCCUUUC |
| 41 | si-16A: | GGACGUUCAAGAGGUGCAA |
| 42 | si-16AS: | UUGCACCUCUUGAACGUCC |
| 43 | si-17A: | GUUCAAGAGGUGCAAUAUA |
| 44 | si-17AS: | UAUAUUGCACCUCUUGAAC |
| 45 | si-18A: | GCAAUAUACAUUUGACCUA |
| 46 | si-18AS: | UAGGUCAAAUGUAUAUUGC |
| 47 | si-19A: | GGUGGUGCUUACGGAGAAA |
| 48 | si-19AS: | UUUCUCCGUAAGCACCACC |
| 49 | si-20A: | GGAGUUAAGAGGAGAAUCA |
| 50 | si-20AS: | UGAUUCUCCUCUUAACUCC |
| 51 | si-21A: | GACCAAAUUCCUUCCUUUA |
| 52 | si-21AS: | UAAAGGAAGGAAUUUGGUC; |

| | | |
|---|---|---|
| in the above nucleic acid sequences, U nucleotides and T nucleotides are interchangeable. | | |

In another preferred embodiment, the present invention provides a nanoliposome composition comprising siRNA as a therapeutically active component.

In another preferred embodiment, the nanoliposome composition comprises: a carrier, the siRNA, a pharmaceutical excipient, and a combination thereof.

In another preferred embodiment, the carrier comprises one or more cationic lipid compounds, helper lipids, structural lipids, polymer conjugated lipids, and combinations thereof.

In another preferred embodiment, the molar ratio of the cationic lipid compound to the helper lipid is 0.5:1 - 15:1.

In another preferred embodiment, the molar ratio of the cationic lipid compound to the structural lipid is 0.5:1 - 5:1.

In another preferred embodiment, the molar ratio of the cationic lipid compound to the polymer conjugated lipid is 10:1 - 250:1.

In another preferred embodiment, the nanoliposome composition is lipid nanoparticles, and the average size of the lipid nanoparticles is 30-200 nm.

In another preferred embodiment, the polydispersity index of the formulation of lipid nanoparticles is ≤ 0.3.

In another preferred embodiment, the pharmaceutical excipient comprises a diluent, stabilizer, preservative, or freeze-drying protectant, and combinations thereof.

In the fifth aspect of the present invention, it provides an shRNA targeting a human TDG sequence, wherein the shRNA has a sequence selected from the group consisting of (each presented 5' to 3'):

| | | |
|---|---|---|
| 53 | sh-1A: | CCGGGAACGAAATATGGACGTTCAACTCGAGTTGA ACGTCCATATTTCGTTCTTTTTG |
| 54 | sh-1AS: | AATTCAAAAAGAACGAAATATGGACGTTCAACTC GAGTTGAACGTCCATATTTCGTTC |
| 55 | sh-2A: | CCGGGGACTTAAGAGATCAGTTGAACTCGAGTTC AACTGATCTCTTAAGTCCTTTTTG |
| 56 | sh-2AS: | AATTCAAAAAGGACTTAAGAGATCAGTTGAACTC GAGTTCAACTGATCTCTTAAGTCC |
| 57 | sh-3A: | CCGGGGTTATGAGGCAGCATATGGTCTCGAGACCA TATGCTGCCTCATAACCTTTTTG |
| 58 | sh-3AS: | AATTCAAAAAGGTTATGAGGCAGCATATGGTCTC GAGACCATATGCTGCCTCATAACC |
| 59 | sh-4A: | CCGGCAGCTATTCCCTTCAGCAACTCGAGTTGCT GAAGGGAATAGCTGTTTTTG |
| 60 | sh-4AS: | AATTCAAAAACAGCTATTCCCTTCAGCAACTCGA GTTGCTGAAGGGAATAGCTG |
| 61 | sh-5A: | CCGGCCAGAACAACAGAACCAAACTCGAGTTTGGTT CTGTTGTTCTGGTTTTTG |
| 62 | sh-5AS: | AATTCAAAAACCAGAACAACAGAACCAAACTCGA GTTTGGTTCTGTTGTTCTGG |
| 63 | sh-6A: | CCGGGGATGATCACACTCTACCACTCGAGTGGTA GAGTGTGATCATCCTTTTTG |
| 64 | sh-6AS: | AATTCAAAAAGGATGATCACACTCTACCACTCGA GTGGTAGAGTGTGATCATCC |
| 65 | sh-7A: | CCGGGCAAAGATCTCTCCAGTAACTCGAGTTACTGG AGAGATCTTTGCTTTTTG |
| 66 | sh-7AS: | AATTCAAAAAGCAAAGATCTCTCCAGTAACTCGAG TTACTGGAGAGATCTTTGC |
| 67 | sh-8A: | CCGGGAAGGAGGACGTATTCTAGTACTCGAGTACTA GAATACGTCCTCCTTCTTTTTG |
| 68 | sh-8AS: | AATTCAAAAAGAAGGAGGACGTATTCTAGTACTCGAG |
| 69 | sh-9A: | TACTAGAATACGTCCTCCTTC CCGGGACGTATTCTAGTACAGAACTCGAGTTCTGTA |
| 70 | sh-9AS: | CTAGAATACGTCTTTTTG AATTCAAAAAGACGTATTCTAGTACAGAACTCGAG |
| 71 | sh-10A: | TTCTGTACTAGAATACGTC CCGGCACGAATAGCAGTGTTTAACTCGAGTTAAACA |
| 72 | sh-10AS: | CTGCTATTCGTGTTTTTG AATTCAAAAACACGAATAGCAGTGTTTAACTCGAG |
| 73 | sh-11A: | TTAAACACTGCTATTCGTG CCGGGAAACTCTCTGCTATGTTACTCGAGTAACA |
| 74 | sh-11AS: | TAGCAGAGAGTTTCTTTTTG AATTCAAAAAGAAACTCTCTGCTATGTTACTCGAG |
| 75 | sh-12A: | TAACATAGCAGAGAGTTTC CCGGGACAAAGTTCATTACTACACTCGAGTGTAGTA |
| 76 | sh-12AS: | ATGAACTTTGTCTTTTTG AATTCAAAAAGACAAAGTTCATTACTACACTCGAGTG |
| 77 | sh-13A: | TAGTAATGAACTTTGTC CCGGGAAGGACTTAAGAGATCAGTTCTCGAGAAC |
| 78 | sh-13AS: | TGATCTCTTAAGTCCTTCTTTTTG AATTCAAAAAGAAGGACTTAAGAGATCAGTTCTCGA |
| 79 | sh-14A: | GAACTGATCTCTTAAGTCCTTC CCGGGACTTAAGAGATCAGTTGACTCGAGTCAACTG |
| 80 | sh-14AS: | ATCTCTTAAGTCTTTTTG AATTCAAAAAGACTTAAGAGATCAGTTGACTCGAGT |
| 81 | sh-15A: | CAACTGATCTCTTAAGTC CCGGGAAAGGCATTGAACGAAATATCTCGAGATATTT |
| 82 | sh-15AS: | CGTTCAATGCCTTTCTTTTTG AATTCAAAAAGAAAGGCATTGAACGAAATATCTC |
| | | GAGATATTTCGTTCAATGCCTTTC |
| 83 | sh-16A: | CCGGGGACGTTCAAGAGGTGCAACTCGAGTTGCAC CTCTTGAACGTCCTTTTTG |
| 84 | sh-16AS: | AATTCAAAAAGGACGTTCAAGAGGTGCAACTCGAG TTGCACCTCTTGAACGTCC |
| 85 | sh-17A: | CCGGGTTCAAGAGGTGCAATATACTCGAGTATATTG CACCTCTTGAACTTTTTG |
| 86 | sh-17AS: | AATTCAAAAAGTTCAAGAGGTGCAATATACTCGA GTATATTGCACCTCTTGAAC |
| 87 | sh-18A: | CCGGGCAATATACATTTGACCTACTCGAGTAGGTC AAATGTATATTGCTTTTTG |
| 88 | sh-18AS: | AATTCAAAAAGCAATATACATTTGACCTACTCGA GTAGGTCAAATGTATATTGC |
| 89 | sh-19A: | CCGGGGTGGTGCTTACGGAGAAACTCGAGTTTCT CCGTAAGCACCACCTTTTTG |
| 90 | sh-19AS: | AATTCAAAAAGGTGGTGCTTACGGAGAAACTCGA GTTTCTCCGTAAGCACCACC |
| 91 | sh-20A: | CCGGGGAGTTAAGAGGAGAATCACTCGAGTGATT CTCCTCTTAACTCCTTTTTG |
| 92 | sh-20AS: | AATTCAAAAAGGAGTTAAGAGGAGAATCACTCGAG TGATTCTCCTCTTAACTCC |
| 93 | sh-21A: | CCGGGACCAAATTCCTTCCTTTACTCGAGTAAAGGA AGGAATTTGGTCTTTTTG |
| 94 | sh-21AS: | AATTCAAAAAGACCAAATTCCTTCCTTTACTCGAG TAAAGGAAGGAATTTGGTC. |

In the sixth aspect of the present invention, it provides an sgRNA sequence for constructing a *Trp53* knockout mouse model, wherein the sequence (5 '-3') comprises:

| | | |
|---|---|---|
| 95 | mTrp53_sg-1: | AGTGAAGCCCTCCGAGTGTC; |
| 96 | mTrp53_sg-2: | GACCCTGTCACCGAGACCCC. |

In the seventh aspect of the present invention, it provides an sgRNA sequence for constructing a *Tdg* knockout mouse model, wherein the sequence comprises:

| | | |
|---|---|---|
| 97 | mTdg_sg-1: | TTCTAGATTGGCATTAACCC; |
| 98 | mTdg_sg-2: | CCGGGAAGGAGGGCGCATCC. |

It should be understood that within the scope of the present invention, the above-mentioned technical features of the present invention and the specific technical features described in the following embodiments can be combined with each other to form new or preferred technical solutions. Due to space limitations, we will not elaborate on each one here.

### BRIEF DESCRIPTION OF DRAWINGS

FIG. 1 shows a study on the development of *TP53* gene deficiency-associated and non-associated tumor cells in *Tdg* gene knockout mice; a: the effect of knocking out *Tdg* (KT for short, the same below) on tumor burden in a mouse lung adenocarcinoma model driven by *Kras^{G12D}* mutation (K for short, the same below); b: the effect of additional *Tdg* knockout (KPT for short, the same below) on tumor burden in a mouse lung cancer model with *Kras^{G12D}* mutation and *Trp53* knockout (KP for short, the same below).
FIG. 2 shows the KEGG pathway enrichment analysis of differentially expressed genes in KPT cells, with the control group consisting of KP cells.
FIG. 3 shows the results of immunofluorescence detection of double stranded RNA in the cytoplasm of KP and KPT cells, with the control group being KP cells treated with 5Aza.
FIG. 4 shows the single-cell sequencing results of KP *Tdg* KO and KP cell subcutaneous tumor models.
FIG. 5 shows the changes in tumor volume (a) and mouse body weight (b) after treatment with PD1 monoclonal antibody and CTLA4 monoclonal antibody in KP and KPT mice, respectively.
FIG. 6: a: the changes in cell proliferation after knocking out *TDG* in the *TP53* deficient human lung adenocarcinoma cell line NCI-H1299; b: the changes in cell proliferation after restoration of wild-type *TP53* in *TDG* knockout NCI-H1299 cells; wherein *TDG* KO-1 and *TDG* KO-2 are two monoclonal clones of *TDG* knockout.
FIG. 7 shows the changes in subcutaneous tumor formation after knocking out *TDG* in the *TP53* deficient human lung adenocarcinoma cell line NCI-H1299; wherein *TDG* KO-1 and *TDG* KO-2 are two monoclonal clones of *TDG* knockout.
FIG. 8 shows the changes in cell proliferation after knocking out *TDG* in SK-MEL-28 (a) with *TP53* mutation and A375 (b) with *TP53* wild-type; wherein *TDG* KO-1 and *TDG* KO-2 are two monoclonal clones of *TDG* knockout.
FIG. 9 shows the B16 model of fully immunized mice. The results showed that double knockout of *Tdg* and *Trp53* significantly inhibited the subcutaneous tumorigenicity of B16, while single knockout of *Tdg* or *Trp53* had no effect. However, further knockout of the key gene Mavs for innate immune activation restored the subcutaneous tumorigenicity ability.
FIG. 10 shows the heatmap analysis of differentially expressed genes in B16 cells, where knocking out Mavs eliminated the effects of *Tdg* and *Trp53* double knockout on innate immune related genes.
FIG. 11 shows the knockdown effect of 21 siRNA targeting different sites of human *TDG* on A549 cells.
FIG. 12 shows the effect of *TDG* knockdown on *TP53* mutation and wild-type tumor cell proliferation.

### DETAILED DESCRIPTION

After long-term and in-depth research, the inventor unexpectedly discovered that downregulating TDG activity or expression can effectively improve the condition of tumors associated with TP53 gene mutations, and when combined with immune checkpoint inhibitors, can further inhibit tumor growth. Based on the above findings, the inventor has completed the present invention.

### Treatment of TP53 gene mutation tumors by downregulating TDG levels

The inventors have discovered that downregulating TDG activity or expression levels can effectively improve the condition of tumors associated with *TP53* gene mutations. Based on this, the inventors have developed a drug targeting *TP53* gene mutation tumors, which may comprise: TDG inhibitors, and pharmaceutically acceptable excipients thereof.

In the present invention, the method for downregulating TDG activity or expression level is not particularly limited, and traditional methods such as small molecule inhibitors or pharmaceutically acceptable salts thereof, peptide drugs, etc. can be used. Preferably, the method for downregulation comprises administering to a subject in need thereof an effective amount of a therapeutic component selected from the group consisting of 6-keto-prostaglandin F1a, prostaglandin A1, E6 berberine, juglone, GW-5074, rottlerin, cefoxime, idarubicin, doxorubicin, menadione, Congo red, sodium ferric gluconate, ferrous sulfate, aurothioglucose, Evans blue, closantel, cinchonine sulfate, hexadimethrine bromide, indigo carmine, protamine chloride, and any combination thereof.

The method may also comprise administering other methods that can be used to downregulate TDG activity or expression levels, such as administering small molecule inhibitors as described in US2022213070A1 and WO2022047288A1.

In other preferred embodiments of the present invention, other techniques known in the art such as CRISPR-Cas9 gene knockout technology or RNA therapeutic active components such as siRNA can also be used. In a preferred embodiment of the present invention, the TDG inhibitor is an sgRNA targeting the human TDG sequence for use in CRISPR-Cas9 gene knockout technology, which can be used for gene editing of tumor cells in vitro using CRISPR-Cas9 gene knockout technology.

Furthermore, the TDG inhibitor is an sgRNA with a nucleotide sequence of (5 '-3'):

| | | |
|---|---|---|
| 1 | sg-1: | GCTATAAAACTGGAAGTTAG |
| 2 | sg-2: | TGTATTTCAGAGGCTCCAAA |
| 3 | sg-3: | ACGAAATATGGACGTTCAAG |
| 4 | sg-4: | TGGGTCATCCACTGCCCATT |
| 5 | sg-5: | TGGCATAAACCCGGGACTAA |
| 6 | sg-6: | TTTGACCTACAGCTTGCCCA |
| 7 | sg-7: | GTTGAGAGCGTGGAGTTAAG |
| 8 | sg-8: | TGAGGTCCAGCTGAACCATA |
| 9 | sg-9: | GGGCATCATTACCCTGGACC |
| 10 | sg-10: | CTACCAGGGAAGTATGGTAT. |

Furthermore, TDG inhibitors are at least one type of siRNA targeting the human TDG sequence for use in RNA interference technology. When the TDG inhibitor is siRNA, it can be delivered to tumor tissues in vivo using drug delivery systems known in the art, such as nanoliposomes.

Furthermore, the TDG inhibitor is siRNA with a nucleotide sequence of (5 '-3'):

| | | |
|---|---|---|
| 11 | si-1S: | GAACGAAAUAUGGACGUUCAA |
| 12 | si-1AS: | UUGAACGUCCAUAUUUCGUUC |
| 13 | si-2S: | GGACUUAAGAGAUCAGUUGAA |
| 14 | si-2AS: | UUCAACUGAUCUCUUAAGUCC |
| 15 | si-3A: | GGUUAUGAGGCAGCAUAUGGU |
| 16 | si-3AS: | ACCAUAUGCUGCCUCAUAACC |
| 17 | si-4A: | CAGCUAUUCCCUUCAGCAA |
| 18 | si-4AS: | UUGCUGAAGGGAAUAGCUG |
| 19 | si-5A: | CCAGAACAACAGAACCAAA |
| 20 | si-5AS: | UUUGGUUCUGUUGUUCUGG |
| 21 | si-6A: | GGAUGAUCACACUCUACCA |
| 22 | si-6AS: | UGGUAGAGUGUGAUCAUCC |
| 23 | si-7A: | GCAAAGAUCUCUCCAGUAA |
| 24 | si-7AS: | UUACUGGAGAGAUCUUUGC |
| 25 | si-8A: | GAAGGAGGACGUAUUCUAGUA |
| 26 | si-8AS: | UACUAGAAUACGUCCUCCUUC |
| 27 | si-9A: | GACGUAUUCUAGUACAGAA |
| 28 | si-9AS: | UUCUGUACUAGAAUACGUC |
| 29 | si-10A: | CACGAAUAGCAGUGUUUAA |
| 30 | si-10AS: | UUAAACACUGCUAUUCGUG |
| 31 | si-11A: | GAAACUCUCUGCUAUGUUA |
| 32 | si-11AS: | UAACAUAGCAGAGAGUUUC |
| 33 | si-12A: | GACAAAGUUCAUUACUACA |
| 34 | si-12AS: | UGUAGUAAUGAACUUUGUC |
| 35 | si-13A: | GAAGGACUUAAGAGAUCAGUU |
| 36 | si-13AS: | AACUGAUCUCUUAAGUCCUUC |
| 37 | si-14A: | GACUUAAGAGAUCAGUUGA |
| 38 | si-14AS: | UCAACUGAUCUCUUAAGUC |
| 39 | si-15A: | GAAAGGCAUUGAACGAAAUAU |
| 40 | si-15AS: | AUAUUUCGUUCAAUGCCUUUC |
| 41 | si-16A: | GGACGUUCAAGAGGUGCAA |
| 42 | si-16AS: | UUGCACCUCUUGAACGUCC |
| 43 | si-17A: | GUUCAAGAGGUGCAAUAUA |
| 44 | si-17AS: | UAUAUUGCACCUCUUGAAC |
| 45 | si-18A: | GCAAUAUACAUUUGACCUA |
| 46 | si-18AS: | UAGGUCAAAUGUAUAUUGC |
| 47 | si-19A: | GGUGGUGCUUACGGAGAAA |
| 48 | si-19AS: | UUUCUCCGUAAGCACCACC |
| 49 | si-20A: | GGAGUUAAGAGGAGAAUCA |
| 50 | si-20AS: | UGAUUCUCCUCUUAACUCC |
| 51 | si-21A: | GACCAAAUUCCUUCCUUUA |
| 52 | si-21AS: | UAAAGGAAGGAAUUUGGUC. |

Furthermore, TDG inhibitors are at least one type of shRNA targeting the human TDG sequence for use in RNA interference technology. When the TDG inhibitor is shRNA, it can be delivered to tumor tissues in vivo using drug delivery systems known in the art, such as nanoliposomes.

Furthermore, the TDG inhibitor is shRNA with a nucleotide sequence of (5 '-3'):

| | | |
|---|---|---|
| 53 | sh-1A: | CCGGGAACGAAATATGGACGTTCAACTCGAGTTGA ACGTCCATATTTCGTTCTTTTTG |
| 54 | sh-1AS: | AATTCAAAAAGAACGAAATATGGACGTTCAACTC |
| | | GAGTTGAACGTCCATATTTCGTTC |
| 55 | sh-2A: | CCGGGGACTTAAGAGATCAGTTGAACTCGAGTTC AACTGATCTCTTAAGTCCTTTTTG |
| 56 | sh-2AS: | AATTCAAAAAGGACTTAAGAGATCAGTTGAACTC GAGTTCAACTGATCTCTTAAGTCC |
| 57 | sh-3A: | CCGGGGTTATGAGGCAGCATATGGTCTCGAGACCA TATGCTGCCTCATAACCTTTTTG |
| 58 | sh-3AS: | AATTCAAAAAGGTTATGAGGCAGCATATGGTCTC GAGACCATATGCTGCCTCATAACC |
| 59 | sh-4A: | CCGGCAGCTATTCCCTTCAGCAACTCGAGTTGCT GAAGGGAATAGCTGTTTTTG |
| 60 | sh-4AS: | AATTCAAAAACAGCTATTCCCTTCAGCAACTCGA GTTGCTGAAGGGAATAGCTG |
| 61 | sh-5A: | CCGGCCAGAACAACAGAACCAAACTCGAGTTTGGTT CTGTTGTTCTGGTTTTTG |
| 62 | sh-5AS: | AATTCAAAAACCAGAACAACAGAACCAAACTCGA GTTTGGTTCTGTTGTTCTGG |
| 63 | sh-6A: | CCGGGGATGATCACACTCTACCACTCGAGTGGTA GAGTGTGATCATCCTTTTTG |
| 64 | sh-6AS: | AATTCAAAAAGGATGATCACACTCTACCACTCGA GTGGTAGAGTGTGATCATCC |
| 65 | sh-7A: | CCGGGCAAAGATCTCTCCAGTAACTCGAGTTACTGG AGAGATCTTTGCTTTTTG |
| 66 | sh-7AS: | AATTCAAAAAGCAAAGATCTCTCCAGTAACTCGAG TTACTGGAGAGATCTTTGC |
| 67 | sh-8A: | CCGGGAAGGAGGACGTATTCTAGTACTCGAGTACTA GAATACGTCCTCCTTCTTTTTG |
| 68 | sh-8AS: | AATTCAAAAAGAAGGAGGACGTATTCTAGTACTCGAG TACTAGAATACGTCCTCCTTC |
| 69 | sh-9A: | CCGGGACGTATTCTAGTACAGAACTCGAGTTCTGTA CTAGAATACGTCTTTTTG |
| 70 | sh-9AS: | AATTCAAAAAGACGTATTCTAGTACAGAACTCGAG TTCTGTACTAGAATACGTC |
| 71 | sh-10A: | CCGGCACGAATAGCAGTGTTTAACTCGAGTTAAACA CTGCTATTCGTGTTTTTG |
| 72 | sh-10AS: | AATTCAAAAACACGAATAGCAGTGTTTAACTCGAG TTAAACACTGCTATTCGTG |
| 73 | sh-11A: | CCGGGAAACTCTCTGCTATGTTACTCGAGTAACA TAGCAGAGAGTTTCTTTTTG |
| 74 | sh-11AS: | AATTCAAAAAGAAACTCTCTGCTATGTTACTCGAG TAACATAGCAGAGAGTTTC |
| 75 | sh-12A: | CCGGGACAAAGTTCATTACTACACTCGAGTGTAGTA ATGAACTTTGTCTTTTTG |
| 76 | sh-12AS: | AATTCAAAAAGACAAAGTTCATTACTACACTCGAGTG TAGTAATGAACTTTGTC |
| 77 | sh-13A: | CCGGGAAGGACTTAAGAGATCAGTTCTCGAGAAC TGATCTCTTAAGTCCTTCTTTTTG |
| 78 | sh-13AS: | AATTCAAAAAGAAGGACTTAAGAGATCAGTTCTCGA GAACTGATCTCTTAAGTCCTTC |
| 79 | sh-14A: | CCGGGACTTAAGAGATCAGTTGACTCGAGTCAACTG ATCTCTTAAGTCTTTTTG |
| 80 | sh-14AS: | AATTCAAAAAGACTTAAGAGATCAGTTGACTCGAGT CAACTGATCTCTTAAGTC |
| 81 | sh-15A: | CCGGGAAAGGCATTGAACGAAATATCTCGAGATATTT CGTTCAATGCCTTTCTTTTTG |
| 82 | sh-15AS: | AATTCAAAAAGAAAGGCATTGAACGAAATATCTC GAGATATTTCGTTCAATGCCTTTC |
| 83 | sh-16A: | CCGGGGACGTTCAAGAGGTGCAACTCGAGTTGCAC |
| | | CTCTTGAACGTCCTTTTTG |
| 84 | sh-16AS: | AATTCAAAAAGGACGTTCAAGAGGTGCAACTCGAG TTGCACCTCTTGAACGTCC |
| 85 | sh-17A: | CCGGGTTCAAGAGGTGCAATATACTCGAGTATATTG CACCTCTTGAACTTTTTG |
| 86 | sh-17AS: | AATTCAAAAAGTTCAAGAGGTGCAATATACTCGA GTATATTGCACCTCTTGAAC |
| 87 | sh-18A: | CCGGGCAATATACATTTGACCTACTCGAGTAGGTC AAATGTATATTGCTTTTTG |
| 88 | sh-18AS: | AATTCAAAAAGCAATATACATTTGACCTACTCGA GTAGGTCAAATGTATATTGC |
| 89 | sh-19A: | CCGGGGTGGTGCTTACGGAGAAACTCGAGTTTCT CCGTAAGCACCACCTTTTTG |
| 90 | sh-19AS: | AATTCAAAAAGGTGGTGCTTACGGAGAAACTCGA GTTTCTCCGTAAGCACCACC |
| 91 | sh-20A: | CCGGGGAGTTAAGAGGAGAATCACTCGAGTGATT CTCCTCTTAACTCCTTTTTG |
| 92 | sh-20AS: | AATTCAAAAAGGAGTTAAGAGGAGAATCACTCGAG TGATTCTCCTCTTAACTCC |
| 93 | sh-21A: | CCGGGACCAAATTCCTTCCTTTACTCGAGTAAAGGA AGGAATTTGGTCTTTTTG |
| 94 | sh-21AS: | AATTCAAAAAGACCAAATTCCTTCCTTTACTCGAG TAAAGGAAGGAATTTGGTC. |

When prepared into nanoliposomes, a preferred embodiment of the nanoliposome composition comprises: a carrier, a loaded drug, a pharmaceutical excipient, and combinations thereof.

Among them, the helper lipid may comprise one or a combination of phosphatidylcholine, phosphatidylethanolamine, sphingomyelin (SM), sterols and derivatives thereof, ceramides, and charged lipids. As a preferred embodiment, phosphatidylcholine comprises: DSPC, DPPC, DMPC, DOPC, POPC. As a preferred embodiment, phosphatidylethanolamine is DOPE. As a preferred embodiment, the sterol is cholesterol. As a preferred embodiment, the charged lipids are DOTAP, DOTMA, and 18PA. This is not an exhaustive list, and any combination of cationic lipid compounds using the structure of the present invention is within the scope of protection of the present invention and is inspired by the present invention. This is not an exhaustive list, and the selection of helper lipids is not limited. Any cationic lipid compound using the structure of the present invention is within the scope of protection of the present invention and is inspired by the present invention.

Charged lipids refer to a type of lipid compound that exists in a positively or negatively charged form; and the charge it carries does not depend on the pH within the physiological range, such as pH 3-9, and is not affected by pH. Charged lipids can be synthesized or naturally derived. Examples of charged lipids include but are not limited to DOTAP, DOTMA, and 18PA.

The siRNA of the present invention may also comprise one or more functional nucleotide analogues, such as pseudouridine, 1-methyl-pseudouridine, or 5-methylcytosine. The examples provided here are not exhaustive, and any modified mRNA or derivatives thereof can be applied to the present invention.

Small molecule compounds can be active components in reagents used for treatment or prevention, such as anti-tumor drugs, anti-infective drugs, local anesthetics, antidepressants, anticonvulsants, antibiotics/antimicrobial agents, antifungal drugs, antiparasitic drugs, hormones, hormone antagonists, immunomodulators, neurotransmitter antagonists, antiglaucoma agents, anesthetics, or imaging agents, etc. This is not an exhaustive list.

Peptides are compounds formed by connecting α-amino acids together through peptide bonds, and are intermediate products of protein hydrolysis.

Proteins are substances with a certain spatial structure formed by the twisting and folding of polypeptide chains composed of amino acids through dehydration condensation. Proteins can be interferons, protein hormones, cytokines, chemokines, or enzymes, etc.

Diluents are any water-soluble excipients known to those skilled in the art that can be used for medicinal purposes, comprising amino acids, monosaccharides, disaccharides, trisaccharides, tetrasaccharides, pentasaccharides, other oligosaccharides, mannitol, dextran, sodium chloride, sorbitol, polyethylene glycol, phosphates, or derivatives thereof, etc.

Stabilizers can be any pharmaceutically acceptable excipients known to those skilled in the art, such as Tween-80, sodium dodecyl sulfate, sodium oleate, mannitol, mannose, or sodium alginate, etc.

Preservatives can be any pharmaceutically acceptable preservatives known to those skilled in the art, such as thiomersal, etc.

Freeze-drying protectants can be any pharmaceutically acceptable freeze-drying protectants known to those skilled in the art, such as: glucose, mannitol, sucrose, lactose, trehalose, maltose, etc.

The TDG inhibitor can inhibit TDG protein expression and activate the innate immune system of tumor patients. In another preferred embodiment of the present invention, the TDG inhibitor may be combined with immune checkpoint inhibitors, such as PD-1 antibody and CTLA-4 antibody, to achieve enhanced therapeutic efficacy.

### Compared with the prior art, the beneficial effects of the present invention are as follows:

The TDG inhibitor used in the present invention can effectively inhibit the expression of human TDG protein, activate the innate immune system of tumor patients, and achieve the effect of killing TP53 mutant tumors.

The present invention will be further explained in conjunction with specific examples. It should be understood that these examples are only used to illustrate the present invention and not to limit the scope of the present invention. The experimental methods without specific conditions specified in the following examples are usually carried out under conventional conditions or conditions recommended by the manufacturer. Unless otherwise specified, percentages and portions are calculated by weight.

### Example 1

Mice with *Kras^{G12D}* mutation and conditional *Tdg* knockout; mice with *Kras^{G12D}* mutation and conditional *Trp53* knockout, and mice with *Kras^{G12D}* mutation and conditional double knockout of both *Trp53* and *Tdg* were bred and obtained. Detailed experimental operation steps could be referred to DuPage *et al.* (2009) "Conditional mouse lung cancer models using adenoviral or lentiviral delivery of Cre recombinase". The sgRNA sequence used for Trp53 knockout in mice in the experiment were: mTrp53_stg-1: AGTGAAGCCCTCCGAGTGTC; mTrp53_sg-2: GACCCTGTCACCGAGACCCC. The sgRNA sequence used for Tdg knockout in mice were: mTd_sg-1: TTCTAGATTGGCATTAACCC; mTdg_sg-2: CCGGGAAGGAGGGCGCATCC. The obtained mice with *Kras^{G12D}* mutation and conditional *Tdg* knockout, and mice with *Kras^{G12D}* mutation and conditional double knockout of both *Trp53* and *Tdg* were utilized. 3-4 weeks after tumor induction via intranasal instillation of Ad-Cre, lung tissues of the mice were collected, perfusion-fixed, and subsequently processed for paraffin embedding, sectioning, and hematoxylin and eosin (H&E) staining.

At 3-4 weeks after tumor induction via intranasal instillation of Ad-Cre, tumor nodules from the loung tissues of mice with *Kras^{G12D}* mutation and conditional *Tdg* knockout, and mice with *Kras^{G12D}* mutation and conditional double knockout of both *Trp53* and *Tdg* were separatedly collected. Two specific cell groups (KP cells and KPT cells) were cultured from these nodules and used for cDNA library construction for RNA-seq.

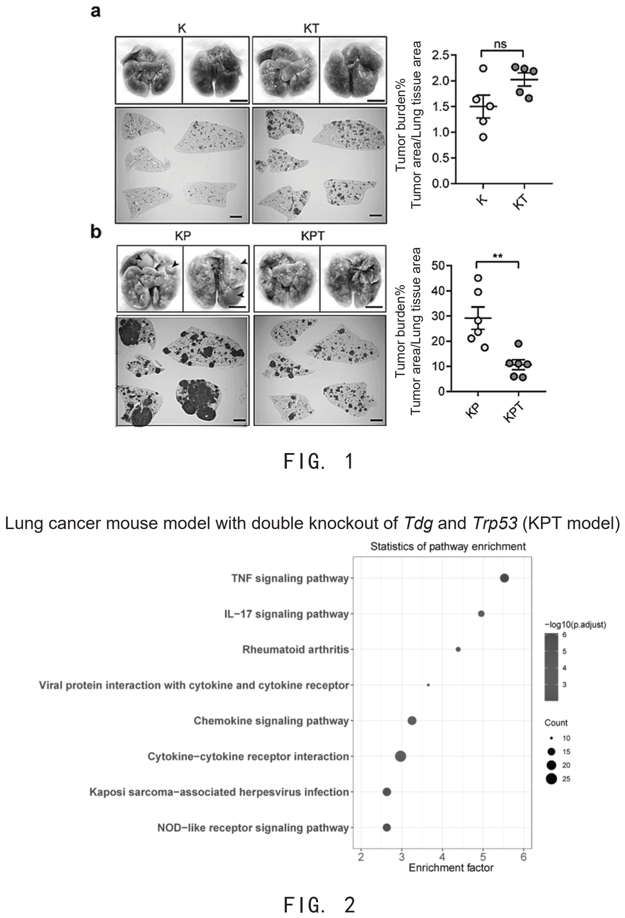

The results showed that in the mouse lung adenocarcinoma model driven by *Kras^{G12D}* mutation with *Trp53* knockout (simulating *TP53* gene deficient tumor cells), further knockout of *Tdg* inhibited tumor growth (FIG. 1b). As a control, in the mouse lung adenocarcinoma model without *Trp53* knockout, *Tdg* knockout alone did not affect tumor growth (FIG. 1a). The above results further confirmed that *Tdg* knockout only exerted an inhibitory effect in the *Trp53* deficient mouse lung adenocarcinoma model. The RNA-seq results showed that pathways related to innate immune activation were significantly enriched after double knockout of *Trp53* and *Tdg* in the mouse lung adenocarcinoma model (FIG. 2), demonstrating that the gene knockout caused tumor cells to mimic a viral infection state and activated innate immune-related signaling pathways.

### Example 2

Immunofluorescence assay was performed to detect double-stranded RNA (dsRNA for short, the same below). Adherent KP and KPT cells were fixed, permeabilized, and blocked. They were then sequentially treated with J2 antibody (specifically recognizes dsRNA, Cat#: SCICONS #10010200), fluorescent secondary antibody Cy^{™}3 (Cat#: Jackson #115-165-146), and DAPI, followed by imaging under a fluorescence microscope.

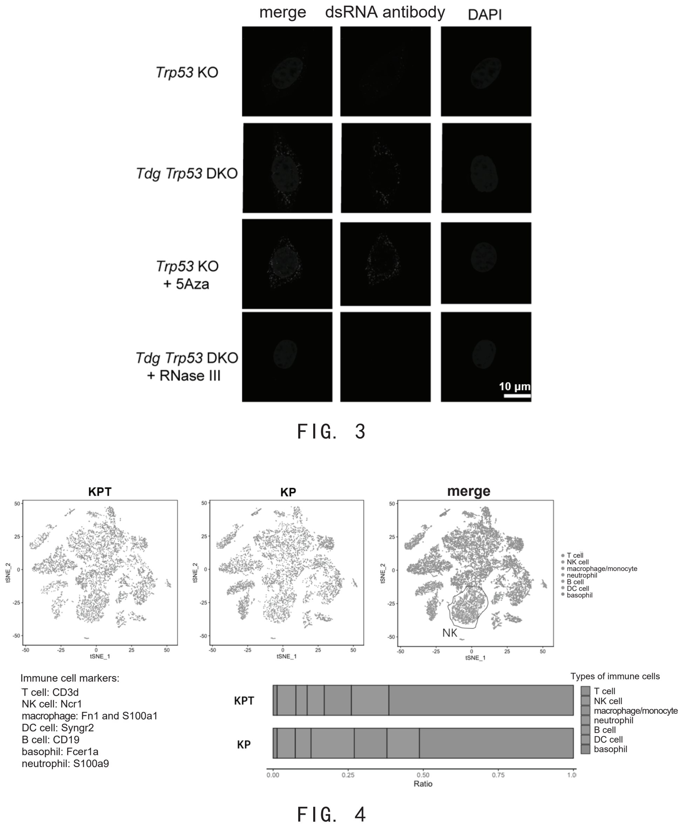

Through immunofluorescence assay, it was detected that compared to KP cells, the level of dsRNA in the cytoplasm of KPT cells (simulating *TDG* inhibition in *TP53*-deficient lung cancer cells) was increased (FIG. 3). KP cells treated with 0.5 µM 5-Aza-2'-deoxycytidine (5Aza) for 5 days were included as a positive experimental control, as literature reports that 5Aza can stimulate dsRNA production in cells. In KPT cells treated with RNase III, the characteristic dsRNA fluorescence signal disappeared, indicating dsRNA degradation by RNase III; this group served as the negative experimental control (FIG. 3).

### Example 3

Single-cell RNA sequencing (scRNA-seq). KP and KPT cells were subcutaneously inoculated to form tumors in two groups of immunocompetent C57 mice, respectively. Cell suspensions were prepared from tumors in the logarithmic growth phase (two weeks post-inoculation). Immune cells infiltrating the tumors were isolated by gradient centrifugation and subjected to scRNA-seq. Immune cells were clustered based on different immune cell markers, and changes in the proportions of various immune cell subsets infiltrating the KP and KPT models were compared.

scRNA-seq results showed that in the KP model (mouse lung adenocarcinoma model with *Kras* G12D mutation and *Trp53* knockout), further knockout of *Tdg* (KP *Tdg* KO model, or KPT model) led to a significant increase in the proportions of T cells and NK cells among tumor-infiltrating lymphocytes in subcutaneous tumors compared to the KP model (FIG. 4).

### Example 4

Effect of combined PD-1 antibody and CTLA-4 antibody treatment on the subcutaneous tumor-forming ability of KP and KPT cells. KP and KPT cells were inoculated into the right thigh of immunocompetent C57 mice, respectively. Drug administration (PD-1 antibody + CTLA-4 antibody, 10+10 mpk) began on day 6, with PBS as the control. Administration was performed twice a week for a total of 5 times. Tumor size and mouse body weight were recorded three times a week. The experiment was terminated when control group tumors approached 2000 mm³, and the mice were euthanized.

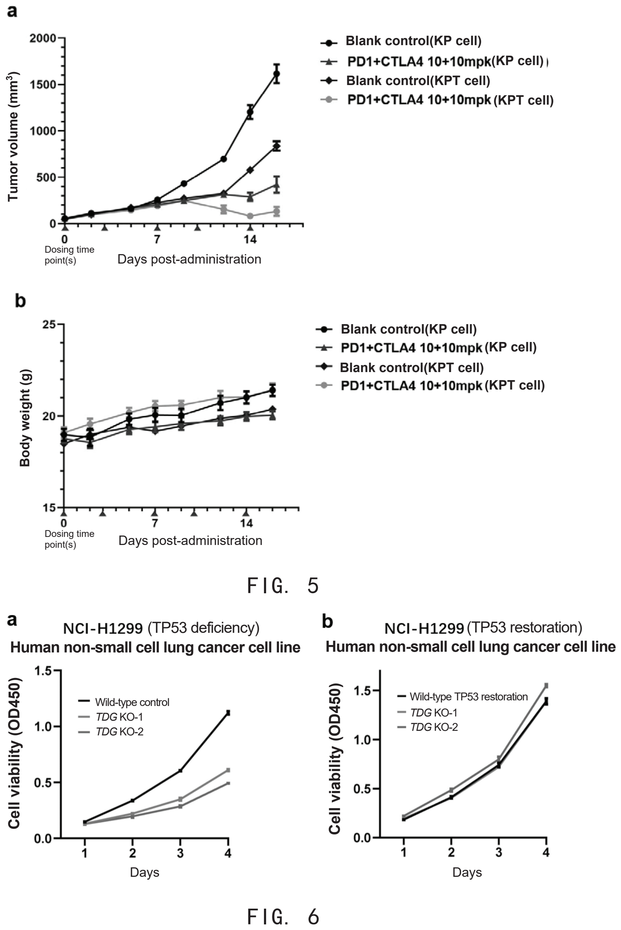

*Tdg* knockout-induced activation of the mouse innate immune system and the increased proportions of T cells and NK cells among tumor-infiltrating lymphocytes in subcutaneous tumors could be combined with PD-1 antibody + CTLA-4 antibody treatment to further inhibit tumor growth (FIG. 5).

### Example 5

Construction of *TDG* knockout human lung cancer cell line NCI-H1299. The pSECC-mediated CRISPR-Cas9 gene editing system was used in the construction of *TDG* knockout human lung cancer cell line NCI-H1299. In this example, pSECC plasmid construction and specific experimental methods were referred to Sanchez-Rivera et al. (2014) "Rapid modelling of cooperating genetic events in cancer through somatic genome editing". Detailed procedures could be referred to the protocol "LentiCRISPRv2 and lentiGuide-Puro: lentiviral CRISPR/Cas9 and single guide RNA". The sgRNAs used for human TDG knockout were: sg-1 to sg-10. TP53 overexpression in NCI-H1299 cells was achieved by integrating the TP53 gene via Lentivirus, using the plasmid pCDH-CMV-3xFLAG-TDG-EF1a-GFP, and GFP-positive cells were sorted by flow cytometry. Cell proliferation assay was performed using a CCK8 kit to detect cell viability. The mouse subcutaneous tumor formation experiment used BALB/c nude mice. Tumor size was recorded starting from day 6 post-cell inoculation, and the experiment was terminated when control group tumors reached approximately 2000 mm³.

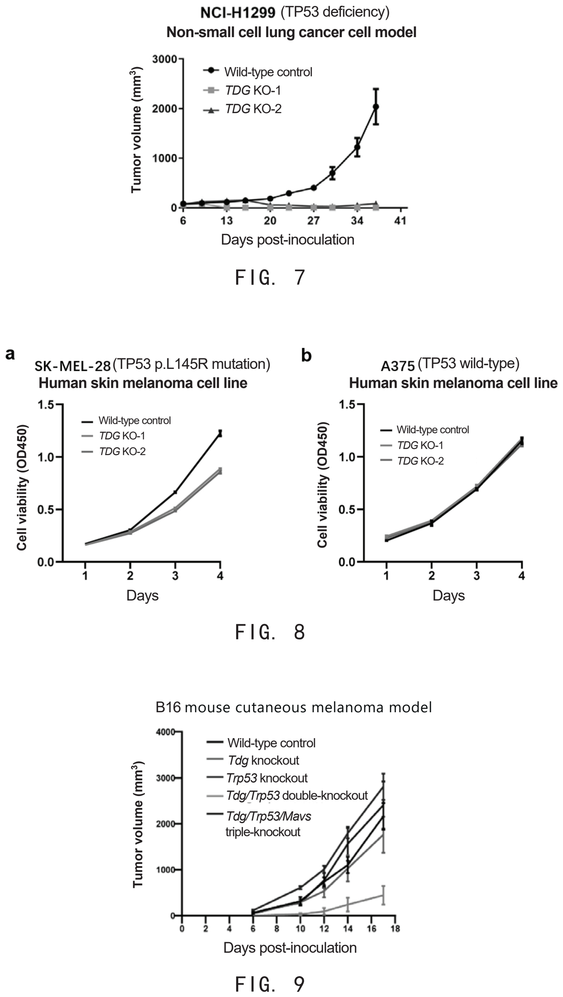

In the *TP53*-deficient human lung adenocarcinoma cell line NCI-H1299, knockout of *TDG* using the CRISPR-Cas9 system resulted in inhibited cell proliferation (FIG. 6a) and in vivo subcutaneous tumor-forming ability (FIG. 7). Restoration of wild-type *TP53* into TDG knockout NCI-H1299 cells restored cell proliferation ability (FIG. 6b). TDG knockout only inhibited the proliferation of *TP53*-deficient NCI-H1299 cells.

### Example 6

Construction of *TDG* knockout human melanoma cell lines SK-MEL-28 and A375. Experimental methods were the same as in Example 5.

The results showed that in the skin melanoma cell line SK-MEL-28 harboring a *TP53* missense mutation (p.L145R), knockout of TDG using the CRISPR-Cas9 system inhibited tumor cell proliferation (FIG. 8a). As a control, the *TP53* wild-type A375 cells were not sensitive to TDG knockout (FIG. 8b).

### Example 7

Effect of *Tdg* knockout, *Trp53* knockout, *Tdg*/*Trp53* double-knockout, and Tdg/Trp53/Mavs triple-knockout on the subcutaneous tumor-forming ability of mouse melanoma cell line B16. The construction method for gene knockout mouse melanoma cell line B16 was the same as in Example 5. The mouse subcutaneous tumor formation experiment used immunocompetent C57 mice. Tumor size was recorded starting from day 6 post-cell inoculation, and the experiment was terminated when control group tumors approached 3000 mm³.

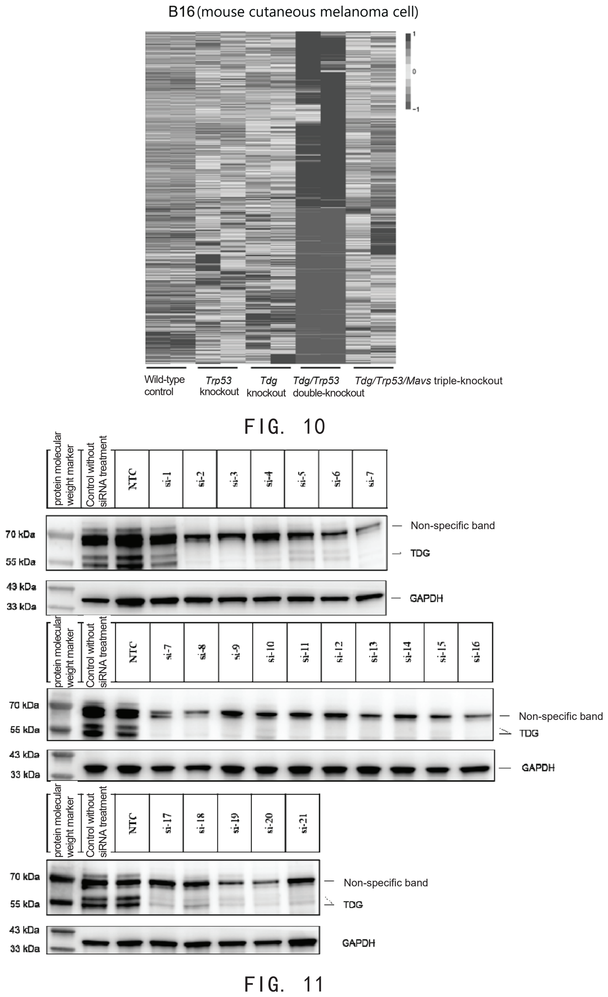

The results showed that in the mouse melanoma cell line B16, knockout of Tdg or Trp53 alone had little effect on subcutaneous tumor formation, while simultaneous knockout of both Tdg and Trp53 greatly reduced its tumor-forming ability (FIG. 9). This model simulated that inhibiting TDG could suppress tumor cell proliferation in TP53-deficient melanoma cells. After double knockout of *Tdg* and *Trp53,* further knockout of Mavs, a key node gene in the innate antiviral immune signaling pathway, completely restored the subcutaneous tumor-forming ability of B16 cells (FIG. 9). Analysis of differentially expressed genes from RNAseq results of different gene knockout B16 cells showed that simultaneous knockout of Tdg and Trp53 significantly affected the expression of innate immune-related genes. After further knockout of Mavs, these gene expression changes were partially reversed (FIG. 10). These results further demonstrated that the mechanism by which Tdg knockout inhibited tumors was achieved through the activation of innate immunity.

### Example 8

21 siRNAs targeting different sites of human TDG were used to knock down TDG in the human non-small cell lung cancer cell line A549. After A549 cells adhered, they were transfected with different siRNAs. Samples were collected 72 hours later, and Western blot confirmed that the siRNAs reduced TDG protein levels to varying degrees (as shown in FIG. 11). The siRNA transfection reagent used was the Lipofectamine RNAiMAX kit (Cat#: Invitrogen #13778), following the manufacturer's instructions. The TDG siRNAs used were: si-1 to si-21. NTC was the negative control, with the sequence: NTC: UUUGUACUACACAAAAGUACUG. For Western blot, the following antibodies were used: the primary against TDG antibody (Cat#: Abcam #ab154192), the secondary antibody (Cat#: Cell signaling #7074), and the primary antibody against GAPDH (Cat#: Cell Signaling #2118).

### Example 9

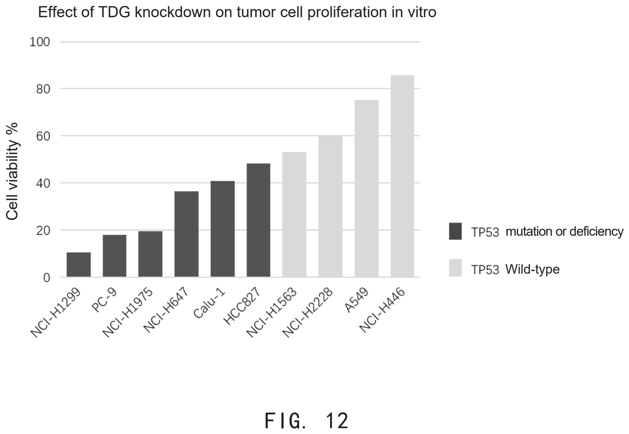

si-2 and si-3, which showed higher TDG knockdown efficiency, could inhibit the in vitro proliferation of various tumor cells (FIG. 12). For tumor cell siRNA transfection, the Lipofectamine RNAiMAX kit (Cat#: Invitrogen #13778) was used, following the manufacturer's instructions. After tumor cells were transfected with siRNA NTC (negative control), si-2, and si-3, respectively, they were cultured for 72 hours. After 72 hours, a second transfection of NTC, si-2, and si-3 was performed, followed by an additional 72-hour culture before cell viability was detected. Cell viability was detected using the CellTiter Glo kit (Cat#: Promega #G7570), following the manufacturer's instructions. Each experiment was performed with six replicates. The cell viability of the NTC group was used as the control (100%) to calculate the relative cell viability (average of the two siRNAs) after siRNA treatment. The results are shown in FIG. 12.

All references mentioned in the present invention are cited as references in this application, as if each reference were cited separately. In addition, it should be understood that after reading the above teaching content of the present invention, those skilled in the art can make various changes or modifications to the present invention, and these equivalent forms also fall within the scope of the claims attached to the present application.

## Claims

1. Use of a TDG inhibitor for the preparation of a pharmaceutical composition for the treatment of tumors associated with TP53 gene mutations;
wherein the pharmaceutical composition comprises a therapeutically effective amount of the TDG inhibitor and pharmaceutically acceptable excipients thereof.

2. The use of claim 1, wherein the TP53 gene mutations are selected from the group consisting of missense mutation, gene deletion, frameshift mutation, splice site mutation, and truncation mutation.

3. The use of claim 1, wherein the TDG inhibitor is an inhibitor targeting the human TDG (GENE ID: 6996).

4. The use of claim 1, wherein the TDG inhibitor is selected from the group consisting of small molecule inhibitors, polypeptide drugs, sgRNA compositions targeting the human TDG for use in CRISPR-Cas9 gene knockout technology, siRNA targeting the human TDG sequence for use in RNA interference technology and combinations thereof, shRNA targeting the human TDG sequence for use in RNA interference technology and combinations thereof.

5. The use of claim 1, wherein the TDG inhibitor is an sgRNA targeting the human TDG sequence for use in CRISPR-Cas9 gene knockout technology, and the sgRNA is selected from the group consisting of (each presented 5' to 3') :
| | | |
|---|---|---|
| 1 | sg-1: | GCTATAAAACTGGAAGTTAG |
| 2 | sg-2: | TGTATTTCAGAGGCTCCAAA |
| 3 | sg-3: | ACGAAATATGGACGTTCAAG |
| 4 | sg-4: | TGGGTCATCCACTGCCCATT |
| 5 | sg-5: | TGGCATAAACCCGGGACTAA |
| 6 | sg-6: | TTTGACCTACAGCTTGCCCA |
| 7 | sg-7: | GTTGAGAGCGTGGAGTTAAG |
| 8 | sg-8: | TGAGGTCCAGCTGAACCATA |
| 9 | sg-9: | GGGCATCATTACCCTGGACC |
| 10 | sg-10: | CTACCAGGGAAGTATGGTAT. |

6. The use of claim 1, wherein the TDG inhibitor is an siRNA targeting the human TDG sequence for use in RNA interference technology, and the siRNA is selected from the group consisting of (each presented 5' to 3'):
| | | |
|---|---|---|
| 11 | si-1S: | GAACGAAAUAUGGACGUUCAA |
| 12 | si-1AS: | UUGAACGUCCAUAUUUCGUUC |
| 13 | si-2S: | GGACUUAAGAGAUCAGUUGAA |
| 14 | si-2AS: | UUCAACUGAUCUCUUAAGUCC |
| 15 | si-3A: | GGUUAUGAGGCAGCAUAUGGU |
| 16 | si-3AS: | ACCAUAUGCUGCCUCAUAACC |
| 17 | si-4A: | CAGCUAUUCCCUUCAGCAA |
| 18 | si-4AS: | UUGCUGAAGGGAAUAGCUG |
| 19 | si-5A: | CCAGAACAACAGAACCAAA |
| 20 | si-5AS: | UUUGGUUCUGUUGUUCUGG |
| 21 | si-6A: | GGAUGAUCACACUCUACCA |
| 22 | si-6AS: | UGGUAGAGUGUGAUCAUCC |
| 23 | si-7A: | GCAAAGAUCUCUCCAGUAA |
| 24 | si-7AS: | UUACUGGAGAGAUCUUUGC |
| 25 | si-8A: | GAAGGAGGACGUAUUCUAGUA |
| 26 | si-8AS: | UACUAGAAUACGUCCUCCUUC |
| 27 | si-9A: | GACGUAUUCUAGUACAGAA |
| 28 | si-9AS: | UUCUGUACUAGAAUACGUC |
| 29 | si-10A: | CACGAAUAGCAGUGUUUAA |
| 30 | si-10AS: | UUAAACACUGCUAUUCGUG |
| 31 | si-11A: | GAAACUCUCUGCUAUGUUA |
| 32 | si-11AS: | UAACAUAGCAGAGAGUUUC |
| 33 | si-12A: | GACAAAGUUCAUUACUACA |
| 34 | si-12AS: | UGUAGUAAUGAACUUUGUC |
| 35 | si-13A: | GAAGGACUUAAGAGAUCAGUU |
| 36 | si-13AS: | AACUGAUCUCUUAAGUCCUUC |
| 37 | si-14A: | GACUUAAGAGAUCAGUUGA |
| 38 | si-14AS: | UCAACUGAUCUCUUAAGUC |
| 39 | si-15A: | GAAAGGCAUUGAACGAAAUAU |
| 40 | si-15AS: | AUAUUUCGUUCAAUGCCUUUC |
| 41 | si-16A: | GGACGUUCAAGAGGUGCAA |
| 42 | si-16AS: | UUGCACCUCUUGAACGUCC |
| 43 | si-17A: | GUUCAAGAGGUGCAAUAUA |
| 44 | si-17AS: | UAUAUUGCACCUCUUGAAC |
| 45 | si-18A: | GCAAUAUACAUUUGACCUA |
| 46 | si-18AS: | UAGGUCAAAUGUAUAUUGC |
| 47 | si-19A: | GGUGGUGCUUACGGAGAAA |
| 48 | si-19AS: | UUUCUCCGUAAGCACCACC |
| 49 | si-20A: | GGAGUUAAGAGGAGAAUCA |
| 50 | si-20AS: | UGAUUCUCCUCUUAACUCC |
| 51 | si-21A: | GACCAAAUUCCUUCCUUUA |
| 52 | si-21AS: | UAAAGGAAGGAAUUUGGUC; |
| | | |
|---|---|---|
| in the above nucleic acid sequences, U nucleotides and T nucleotides are interchangeable. | | |

7. The use of claim 1, wherein the TDG inhibitor is an shRNA targeting the human TDG sequence for use in RNA interference technology, and the shRNA is selected from the group consisting of (each presented 5' to 3'):
| | | |
|---|---|---|
| 53 | sh-1A: | CCGGGAACGAAATATGGACGTTCAACTCGAGTTGA ACGTCCATATTTCGTTCTTTTTG |
| 54 | sh-1AS: | AATTCAAAAAGAACGAAATATGGACGTTCAACTC GAGTTGAACGTCCATATTTCGTTC |
| 55 | sh-2A: | CCGGGGACTTAAGAGATCAGTTGAACTCGAGTTC AACTGATCTCTTAAGTCCTTTTTG |
| 56 | sh-2AS: | AATTCAAAAAGGACTTAAGAGATCAGTTGAACTC GAGTTCAACTGATCTCTTAAGTCC |
| 57 | sh-3A: | CCGGGGTTATGAGGCAGCATATGGTCTCGAGACCA |
| | | TATGCTGCCTCATAACCTTTTTG |
| 58 | sh-3AS: | AATTCAAAAAGGTTATGAGGCAGCATATGGTCTC GAGACCATATGCTGCCTCATAACC |
| 59 | sh-4A: | CCGGCAGCTATTCCCTTCAGCAACTCGAGTTGCT GAAGGGAATAGCTGTTTTTG |
| 60 | sh-4AS: | AATTCAAAAACAGCTATTCCCTTCAGCAACTCGA GTTGCTGAAGGGAATAGCTG |
| 61 | sh-5A: | CCGGCCAGAACAACAGAACCAAACTCGAGTTTGGTT CTGTTGTTCTGGTTTTTG |
| 62 | sh-5AS: | AATTCAAAAACCAGAACAACAGAACCAAACTCGA GTTTGGTTCTGTTGTTCTGG |
| 63 | sh-6A: | CCGGGGATGATCACACTCTACCACTCGAGTGGTA GAGTGTGATCATCCTTTTTG |
| 64 | sh-6AS: | AATTCAAAAAGGATGATCACACTCTACCACTCGA GTGGTAGAGTGTGATCATCC |
| 65 | sh-7A: | CCGGGCAAAGATCTCTCCAGTAACTCGAGTTACTGG AGAGATCTTTGCTTTTTG |
| 66 | sh-7AS: | AATTCAAAAAGCAAAGATCTCTCCAGTAACTCGAG TTACTGGAGAGATCTTTGC |
| 67 | sh-8A: | CCGGGAAGGAGGACGTATTCTAGTACTCGAGTACTA GAATACGTCCTCCTTCTTTTTG |
| 68 | sh-8AS: | AATTCAAAAAGAAGGAGGACGTATTCTAGTACTCGAG TACTAGAATACGTCCTCCTTC |
| 69 | sh-9A: | CCGGGACGTATTCTAGTACAGAACTCGAGTTCTGTA CTAGAATACGTCTTTTTG |
| 70 | sh-9AS: | AATTCAAAAAGACGTATTCTAGTACAGAACTCGAG TTCTGTACTAGAATACGTC |
| 71 | sh-10A: | CCGGCACGAATAGCAGTGTTTAACTCGAGTTAAACA CTGCTATTCGTGTTTTTG |
| 72 | sh-10AS: | AATTCAAAAACACGAATAGCAGTGTTTAACTCGAG TTAAACACTGCTATTCGTG |
| 73 | sh-11A: | CCGGGAAACTCTCTGCTATGTTACTCGAGTAACA TAGCAGAGAGTTTCTTTTTG |
| 74 | sh-11AS: | AATTCAAAAAGAAACTCTCTGCTATGTTACTCGAG TAACATAGCAGAGAGTTTC |
| 75 | sh-12A: | CCGGGACAAAGTTCATTACTACACTCGAGTGTAGTA ATGAACTTTGTCTTTTTG |
| 76 | sh-12AS: | AATTCAAAAAGACAAAGTTCATTACTACACTCGAGTG TAGTAATGAACTTTGTC |
| 77 | sh-13A: | CCGGGAAGGACTTAAGAGATCAGTTCTCGAGAAC TGATCTCTTAAGTCCTTCTTTTTG |
| 78 | sh-13AS: | AATTCAAAAAGAAGGACTTAAGAGATCAGTTCTCGA GAACTGATCTCTTAAGTCCTTC |
| 79 | sh-14A: | CCGGGACTTAAGAGATCAGTTGACTCGAGTCAACTG ATCTCTTAAGTCTTTTTG |
| 80 | sh-14AS: | AATTCAAAAAGACTTAAGAGATCAGTTGACTCGAGT CAACTGATCTCTTAAGTC |
| 81 | sh-15A: | CCGGGAAAGGCATTGAACGAAATATCTCGAGATATTT CGTTCAATGCCTTTCTTTTTG |
| 82 | sh-15AS: | AATTCAAAAAGAAAGGCATTGAACGAAATATCTC GAGATATTTCGTTCAATGCCTTTC |
| 83 | sh-16A: | CCGGGGACGTTCAAGAGGTGCAACTCGAGTTGCAC CTCTTGAACGTCCTTTTTG |
| 84 | sh-16AS: | AATTCAAAAAGGACGTTCAAGAGGTGCAACTCGAG TTGCACCTCTTGAACGTCC |
| 85 | sh-17A: | CCGGGTTCAAGAGGTGCAATATACTCGAGTATATTG CACCTCTTGAACTTTTTG |
| 86 | sh-17AS: | AATTCAAAAAGTTCAAGAGGTGCAATATACTCGA |
| | | GTATATTGCACCTCTTGAAC |
| 87 | sh-18A: | CCGGGCAATATACATTTGACCTACTCGAGTAGGTC AAATGTATATTGCTTTTTG |
| 88 | sh-18AS: | AATTCAAAAAGCAATATACATTTGACCTACTCGA GTAGGTCAAATGTATATTGC |
| 89 | sh-19A: | CCGGGGTGGTGCTTACGGAGAAACTCGAGTTTCT CCGTAAGCACCACCTTTTTG |
| 90 | sh-19AS: | AATTCAAAAAGGTGGTGCTTACGGAGAAACTCGA GTTTCTCCGTAAGCACCACC |
| 91 | sh-20A: | CCGGGGAGTTAAGAGGAGAATCACTCGAGTGATT CTCCTCTTAACTCCTTTTTG |
| 92 | sh-20AS: | AATTCAAAAAGGAGTTAAGAGGAGAATCACTCGAG TGATTCTCCTCTTAACTCC |
| 93 | sh-21A: | CCGGGACCAAATTCCTTCCTTTACTCGAGTAAAGGA AGGAATTTGGTCTTTTTG |
| 94 | sh-21AS: | AATTCAAAAAGACCAAATTCCTTCCTTTACTCGAG TAAAGGAAGGAATTTGGTC. |

8. The use of claim 1, wherein the pharmaceutical composition further comprises a second therapeutically active component that is an immune checkpoint inhibitor.

9. A pharmaceutical combination comprising:
(1) a first therapeutically active component that is a TDG inhibitor;
(2) a second therapeutically active component that is an immune checkpoint inhibitor.

10. An sgRNA targeting the human TDG sequence for use in CRISPR-Cas9 gene knockout technology, wherein the sgRNA is selected from the group consisting of (each presented 5' to 3'):
| | | |
|---|---|---|
| 1 | sg-1: | GCTATAAAACTGGAAGTTAG |
| 2 | sg-2: | TGTATTTCAGAGGCTCCAAA |
| 3 | sg-3: | ACGAAATATGGACGTTCAAG |
| 4 | sg-4: | TGGGTCATCCACTGCCCATT |
| 5 | sg-5: | TGGCATAAACCCGGGACTAA |
| 6 | sg-6: | TTTGACCTACAGCTTGCCCA |
| 7 | sg-7: | GTTGAGAGCGTGGAGTTAAG |
| 8 | sg-8: | TGAGGTCCAGCTGAACCATA |
| 9 | sg-9: | GGGCATCATTACCCTGGACC |
| 10 | sg-10: | CTACCAGGGAAGTATGGTAT. |

11. An siRNA targeting the human TDG sequence, wherein the siRNA has a sequence selected from the group consisting of (each presented 5' to 3'):
| | | |
|---|---|---|
| 11 | si-1S: | GAACGAAAUAUGGACGUUCAA |
| 12 | si-1AS: | UUGAACGUCCAUAUUUCGUUC |
| 13 | si-2S: | GGACUUAAGAGAUCAGUUGAA |
| 14 | si-2AS: | UUCAACUGAUCUCUUAAGUCC |
| 15 | si-3A: | GGUUAUGAGGCAGCAUAUGGU |
| 16 | si-3AS: | ACCAUAUGCUGCCUCAUAACC |
| 17 | si-4A: | CAGCUAUUCCCUUCAGCAA |
| 18 | si-4AS: | UUGCUGAAGGGAAUAGCUG |
| 19 | si-5A: | CCAGAACAACAGAACCAAA |
| 20 | si-5AS: | UUUGGUUCUGUUGUUCUGG |
| 21 | si-6A: | GGAUGAUCACACUCUACCA |
| 22 | si-6AS: | UGGUAGAGUGUGAUCAUCC |
| 23 | si-7A: | GCAAAGAUCUCUCCAGUAA |
| 24 | si-7AS: | UUACUGGAGAGAUCUUUGC |
| 25 | si-8A: | GAAGGAGGACGUAUUCUAGUA |
| 26 | si-8AS: | UACUAGAAUACGUCCUCCUUC |
| 27 | si-9A: | GACGUAUUCUAGUACAGAA |
| 28 | si-9AS: | UUCUGUACUAGAAUACGUC |
| 29 | si-10A: | CACGAAUAGCAGUGUUUAA |
| 30 | si-10AS: | UUAAACACUGCUAUUCGUG |
| 31 | si-11A: | GAAACUCUCUGCUAUGUUA |
| 32 | si-11AS: | UAACAUAGCAGAGAGUUUC |
| 33 | si-12A: | GACAAAGUUCAUUACUACA |
| 34 | si-12AS: | UGUAGUAAUGAACUUUGUC |
| 35 | si-13A: | GAAGGACUUAAGAGAUCAGUU |
| 36 | si-13AS: | AACUGAUCUCUUAAGUCCUUC |
| 37 | si-14A: | GACUUAAGAGAUCAGUUGA |
| 38 | si-14AS: | UCAACUGAUCUCUUAAGUC |
| 39 | si-15A: | GAAAGGCAUUGAACGAAAUAU |
| 40 | si-15AS: | AUAUUUCGUUCAAUGCCUUUC |
| 41 | si-16A: | GGACGUUCAAGAGGUGCAA |
| 42 | si-16AS: | UUGCACCUCUUGAACGUCC |
| 43 | si-17A: | GUUCAAGAGGUGCAAUAUA |
| 44 | si-17AS: | UAUAUUGCACCUCUUGAAC |
| 45 | si-18A: | GCAAUAUACAUUUGACCUA |
| 46 | si-18AS: | UAGGUCAAAUGUAUAUUGC |
| 47 | si-19A: | GGUGGUGCUUACGGAGAAA |
| 48 | si-19AS: | UUUCUCCGUAAGCACCACC |
| 49 | si-20A: | GGAGUUAAGAGGAGAAUCA |
| 50 | si-20AS: | UGAUUCUCCUCUUAACUCC |
| 51 | si-21A: | GACCAAAUUCCUUCCUUUA |
| 52 | si-21AS: | UAAAGGAAGGAAUUUGGUC; |
| | | |
|---|---|---|
| in the above nucleic acid sequences, U nucleotides and T nucleotides are interchangeable. | | |

12. An shRNA targeting the human TDG sequence, wherein the shRNA has a sequence selected from the group consisting of (each presented 5' to 3'):
| | | |
|---|---|---|
| 53 | sh-1A: | CCGGGAACGAAATATGGACGTTCAACTCGAGTTGA ACGTCCATATTTCGTTCTTTTTG |
| 54 | sh-1AS: | AATTCAAAAAGAACGAAATATGGACGTTCAACTC GAGTTGAACGTCCATATTTCGTTC |
| 55 | sh-2A: | CCGGGGACTTAAGAGATCAGTTGAACTCGAGTTC AACTGATCTCTTAAGTCCTTTTTG |
| 56 | sh-2AS: | AATTCAAAAAGGACTTAAGAGATCAGTTGAACTC GAGTTCAACTGATCTCTTAAGTCC |
| 57 | sh-3A: | CCGGGGTTATGAGGCAGCATATGGTCTCGAGACCA TATGCTGCCTCATAACCTTTTTG |
| 58 | sh-3AS: | AATTCAAAAAGGTTATGAGGCAGCATATGGTCTC GAGACCATATGCTGCCTCATAACC |
| 59 | sh-4A: | CCGGCAGCTATTCCCTTCAGCAACTCGAGTTGCT GAAGGGAATAGCTGTTTTTG |
| 60 | sh-4AS: | AATTCAAAAACAGCTATTCCCTTCAGCAACTCGA GTTGCTGAAGGGAATAGCTG |
| 61 | sh-5A: | CCGGCCAGAACAACAGAACCAAACTCGAGTTTGGTT CTGTTGTTCTGGTTTTTG |
| 62 | sh-5AS: | AATTCAAAAACCAGAACAACAGAACCAAACTCGA GTTTGGTTCTGTTGTTCTGG |
| 63 | sh-6A: | CCGGGGATGATCACACTCTACCACTCGAGTGGTA GAGTGTGATCATCCTTTTTG |
| 64 | sh-6AS: | AATTCAAAAAGGATGATCACACTCTACCACTCGA GTGGTAGAGTGTGATCATCC |
| 65 | sh-7A: | CCGGGCAAAGATCTCTCCAGTAACTCGAGTTACTGG AGAGATCTTTGCTTTTTG |
| 66 | sh-7AS: | AATTCAAAAAGCAAAGATCTCTCCAGTAACTCGAG TTACTGGAGAGATCTTTGC |
| 67 | sh-8A: | CCGGGAAGGAGGACGTATTCTAGTACTCGAGTACTA GAATACGTCCTCCTTCTTTTTG |
| 68 | sh-8AS: | AATTCAAAAAGAAGGAGGACGTATTCTAGTACTCGAG |
| | | TACTAGAATACGTCCTCCTTC |
| 69 | sh-9A: | CCGGGACGTATTCTAGTACAGAACTCGAGTTCTGTA CTAGAATACGTCTTTTTG |
| 70 | sh-9AS: | AATTCAAAAAGACGTATTCTAGTACAGAACTCGAG TTCTGTACTAGAATACGTC |
| 71 | sh-10A: | CCGGCACGAATAGCAGTGTTTAACTCGAGTTAAACA CTGCTATTCGTGTTTTTG |
| 72 | sh-10AS: | AATTCAAAAACACGAATAGCAGTGTTTAACTCGAG TTAAACACTGCTATTCGTG |
| 73 | sh-11A: | CCGGGAAACTCTCTGCTATGTTACTCGAGTAACA TAGCAGAGAGTTTCTTTTTG |
| 74 | sh-11AS: | AATTCAAAAAGAAACTCTCTGCTATGTTACTCGAG TAACATAGCAGAGAGTTTC |
| 75 | sh-12A: | CCGGGACAAAGTTCATTACTACACTCGAGTGTAGTA ATGAACTTTGTCTTTTTG |
| 76 | sh-12AS: | AATTCAAAAAGACAAAGTTCATTACTACACTCGAGTG TAGTAATGAACTTTGTC |
| 77 | sh-13A: | CCGGGAAGGACTTAAGAGATCAGTTCTCGAGAAC TGATCTCTTAAGTCCTTCTTTTTG |
| 78 | sh-13AS: | AATTCAAAAAGAAGGACTTAAGAGATCAGTTCTCGA GAACTGATCTCTTAAGTCCTTC |
| 79 | sh-14A: | CCGGGACTTAAGAGATCAGTTGACTCGAGTCAACTG ATCTCTTAAGTCTTTTTG |
| 80 | sh-14AS: | AATTCAAAAAGACTTAAGAGATCAGTTGACTCGAGT CAACTGATCTCTTAAGTC |
| 81 | sh-15A: | CCGGGAAAGGCATTGAACGAAATATCTCGAGATATTT CGTTCAATGCCTTTCTTTTTG |
| 82 | sh-15AS: | AATTCAAAAAGAAAGGCATTGAACGAAATATCTC GAGATATTTCGTTCAATGCCTTTC |
| 83 | sh-16A: | CCGGGGACGTTCAAGAGGTGCAACTCGAGTTGCAC CTCTTGAACGTCCTTTTTG |
| 84 | sh-16AS: | AATTCAAAAAGGACGTTCAAGAGGTGCAACTCGAG TTGCACCTCTTGAACGTCC |
| 85 | sh-17A: | CCGGGTTCAAGAGGTGCAATATACTCGAGTATATTG CACCTCTTGAACTTTTTG |
| 86 | sh-17AS: | AATTCAAAAAGTTCAAGAGGTGCAATATACTCGA GTATATTGCACCTCTTGAAC |
| 87 | sh-18A: | CCGGGCAATATACATTTGACCTACTCGAGTAGGTC AAATGTATATTGCTTTTTG |
| 88 | sh-18AS: | AATTCAAAAAGCAATATACATTTGACCTACTCGA GTAGGTCAAATGTATATTGC |
| 89 | sh-19A: | CCGGGGTGGTGCTTACGGAGAAACTCGAGTTTCT CCGTAAGCACCACCTTTTTG |
| 90 | sh-19AS: | AATTCAAAAAGGTGGTGCTTACGGAGAAACTCGA GTTTCTCCGTAAGCACCACC |
| 91 | sh-20A: | CCGGGGAGTTAAGAGGAGAATCACTCGAGTGATT CTCCTCTTAACTCCTTTTTG |
| 92 | sh-20AS: | AATTCAAAAAGGAGTTAAGAGGAGAATCACTCGAG TGATTCTCCTCTTAACTCC |
| 93 | sh-21A: | CCGGGACCAAATTCCTTCCTTTACTCGAGTAAAGGA AGGAATTTGGTCTTTTTG |
| 94 | sh-21AS: | AATTCAAAAAGACCAAATTCCTTCCTTTACTCGAG TAAAGGAAGGAATTTGGTC. |

13. An sgRNA sequence for constructing a Trp53 knockout mouse model, wherein the sequence (5 '-3') comprises:
| | | |
|---|---|---|
| 95 | mTrp53_sg-1: | AGTGAAGCCCTCCGAGTGTC; |
| 96 | mTrp53_sg-2: | GACCCTGTCACCGAGACCCC. |

14. An sgRNA sequence for constructing a Tdg knockout mouse model, wherein the sequence comprises:
| | | |
|---|---|---|
| 97 | mTdg_sg-1: | TTCTAGATTGGCATTAACCC; |
| 98 | mTdg_sg-2: | CCGGGAAGGAGGGCGCATCC. |
